# EUROPEAN PATENT APPLICATION

(11) **EP 4 371 487 A1**
(43) Date of publication of application: **22.05.2024**
(21) Application number: 22858753.1
(22) Date of filing: 17.08.2022
(51) Int. Cl.: A61B 5/327, A61B 5/346, A61B 5/28, A61B 5/00, G16H 50/20, G16H 50/30

(54) **METHOD FOR GENERATING MULTIPLE PIECES OF STANDARD ELECTROCARDIOGRAM DATA ON BASIS OF DEEP LEARNING ALGORITHM**

(30) Priority: 17.08.2021 KR 20210107774
(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012290
(87) International publication number: WO 2023/022516

(57) **Abstract**

The present invention relate to a method for generating a plurality of pieces of standard electrocardiogram data based on a deep learning algorithm. According to the present invention, there is provided a method for generating a plurality of pieces of standard electrocardiogram data, the method including: collecting standard 12-lead electrocardiogram data, and classifying the collected standard 12-lead electrocardiogram data into a plurality of styles according to characteristics of a measurement target subject and a specific method; training an electrocardiogram generation model to generate a plurality of lead electrocardiograms by inputting the electrocardiogram data, classified into the plurality of styles, to the electrocardiogram generation model; receiving single- or 2-lead electrocardiogram data measured from the measurement target subject; and generating a plurality of lead electrocardiograms by inputting the single- or 2-lead electrocardiogram data to the electrocardiogram generation model.

## Description

### Technical Field

The present invention relates to a method for generating a plurality of pieces of standard electrocardiogram data based on a deep learning algorithm, and more particularly to a method of generating a plurality of pieces of standard electrocardiogram data from single- or 2-lead information by using a style conversion-based algorithm.

### Background Art

An electrocardiogram is a graphical record of electrical potentials related to heartbeat on the surface of the human body. Such electrocardiograms include not only standard 12-lead electrocardiograms but also exercise stress electrocardiograms and Holter electrocardiograms. Electrocardiograms are used for the examination and diagnosis of circulatory diseases, and have the advantages of being simple, relatively inexpensive, non-invasive, and easily recorded repeatedly.

As for a standard 12-lead electrocardiogram used in hospitals, six electrodes are attached to the front of the chest, three electrodes (four electrodes when a ground electrode is included) are attached to the limbs, 12-lead information is all collected and combined, and then a disease is diagnosed. A 12-lead electrocardiogram records electrical potentials of the heart in 12 electrical directions centered on the heart. Through this, the disease of the heart confined to one area can be accurately diagnosed.

However, it is difficult to measure a 12-lead electrocardiogram at home or in everyday life because the chest must be exposed to attach chest electrodes to take a 12-lead electrocardiogram and it is difficult for the general public to attach nine electrodes (three limb electrodes and six chest electrodes) to correct locations. In addition, it is difficult to use a 12-lead electrocardiogram for real-time monitoring because it is difficult to move after the attachment of ten electrodes.

Recently, there has been proposed a technology for measuring the side leads of a plurality of electrocardiograms using a 1-lead electrocardiogram device such as a smart watch. However, when electrocardiograms are measured using the 1-lead electrocardiogram device, a problem arises in that the accurate state of the heart cannot be determined because the electrocardiograms are not measured at the same time.

A background technology of the present invention is disclosed in Korean Patent Application Publication No. 10-2022-0008447 (published on January 21, 2022).

### Disclosure

### Technical Problem

As described above, according to the present invention, there is provided a method of generating a plurality of pieces of standard electrocardiogram data from single- or 2-lead information by using a style conversion-based algorithm.

### Technical Solution

According to an embodiment of the present invention for overcoming the above technical problem, there is provided a method for generating a plurality of pieces of standard electrocardiogram data by using a standard electrocardiogram generation apparatus, the method including: collecting standard 12-lead electrocardiogram data, and classifying the collected standard 12-lead electrocardiogram data into a plurality of styles according to characteristics of a measurement target subject and a specific method; training an electrocardiogram generation model to generate a plurality of lead electrocardiograms by inputting the electrocardiogram data, classified into the plurality of styles, to the electrocardiogram generation model; receiving single- or 2-lead electrocardiogram data measured from the measurement target subject; and generating a plurality of lead electrocardiograms by inputting the single- or 2-lead electrocardiogram data to the electrocardiogram generation model.

The method may further include training an electrocardiogram classification model to determine the lead type of electrocardiogram to which input electrocardiogram data corresponds by inputting the electrocardiogram data, classified into the plurality of styles, to the electrocardiogram classification model.

Classifying the collected standard 12-lead electrocardiogram data into the plurality of styles may include collecting electrocardiogram data, style information indicating the lead type of electrocardiogram to which the electrocardiogram data corresponds, individual information indicating an individual's body and mental conditions, and health condition information.

Training the electrocardiogram classification model to determine the lead type of electrocardiogram may include extracting the features of lead electrocardiograms by using a plurality of pieces of classified electrocardiogram data and style information corresponding to the plurality of pieces of classified electrocardiogram data, and then training the electrocardiogram classification model by using the electrocardiogram data as input data and the extracted features as output data.

Generating the plurality of lead electrocardiograms may include constructing an electrocardiogram generation model obtained by mixing a generative adversarial network and an autoencoder method, and training the constructed electrocardiogram generation model by conditionally inputting random variables and indices for electrocardiograms to the constructed electrocardiogram generation model.

Generating the plurality of lead electrocardiograms may include extracting features of input electrocardiogram data by inputting input electrocardiogram data or 2-lead electrocardiogram data to the electrocardiogram classification model, and determining which of standard 12-lead electrocardiograms is the input lead electrocardiogram data or 2-lead electrocardiogram data according to the extracted features.

Generating the plurality of lead electrocardiograms may include generating a plurality of electrocardiograms of the remaining styles exclusive of a corresponding lead electrocardiogram by inputting the results of the determination and lead electrocardiogram data or 2-lead electrocardiogram data to the electrocardiogram generation model.

The electrocardiogram classification model and the electrocardiogram generation model may be the same learning models or differently constructed learning models.

### Advantageous Effects

According to the present invention described above, a plurality of pieces of standard electrocardiogram information are generated from 2-lead electrocardiogram information by using a deep learning algorithm, so that the highly accurate electrocardiograms that are not affected by a measurement target subject's age, gender, presence/absence of disease, and electrode attachment locations can be provided.

### Description of Drawings

FIG. 1 is a block diagram of a standard electrocardiogram generation apparatus according to an embodiment of the present invention;
FIG. 2 is a flowchart illustrating a method for generating a plurality of pieces of standard electrocardiogram data by using the standard electrocardiogram generation apparatus according to an embodiment of the present invention; and
FIG. 3 is an example diagram illustrating step S250 shown in FIG. 2.

### Mode for Invention

Preferred embodiments according to the present invention will be described in detail below with reference to the accompanying drawings. In this process, the thicknesses of lines or sizes of components shown in the drawings may be shown exaggerated for clarity and convenience of description.

Additionally, the terms to be described below are terms defined by taking into consideration the functions thereof in the present invention, and may vary depending on the intention or custom of a user or operator. Accordingly, the definitions of these terms should be made based on the content throughout the present specification.

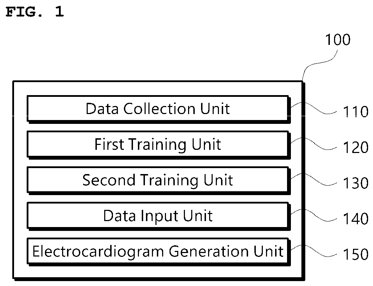

A standard electrocardiogram generation apparatus according to an embodiment of the present invention will be described in detail by using FIG. 1.

FIG. 1 is a block diagram of a standard electrocardiogram generation apparatus according to an embodiment of the present invention.

As shown in FIG. 1, a standard electrocardiogram generation apparatus 100 according to an embodiment of the present invention includes a data collection unit 110, a first training unit 120, a second training unit 130, a data input unit 140, and an electrocardiogram generation unit 150.

First, the data collection unit 110 collects electrocardiogram data. In this case, the electrocardiogram data that is collected is 12-lead electrocardiogram data.

The first training unit 120 constructs an electrocardiogram classification model, and trains the electrocardiogram classification model to classify input lead electrocardiogram by inputting the collected electrocardiogram data to the electrocardiogram classification model.

The second training unit 130 constructs an electrocardiogram generation model, trains the electrocardiogram generation model on the features of electrocardiogram data by inputting the collected electrocardiogram data to the constructed electrocardiogram generation model, and trains the electrocardiogram generation model to generate a plurality of pieces of standard electrocardiogram data by using the learned features.

The data input unit 140 receives single-lead electrocardiogram data or 2-lead electrocardiogram data from a 1-lead electrocardiogram device (not shown).

Finally, the electrocardiogram generation unit 150 generates a plurality of pieces of standard electrocardiogram data exclusive of input electrocardiogram data by inputting the electrocardiogram data of a measurement target subject, received from the data input unit 140, to the trained electrocardiogram classification model and the trained electrocardiogram generation model.

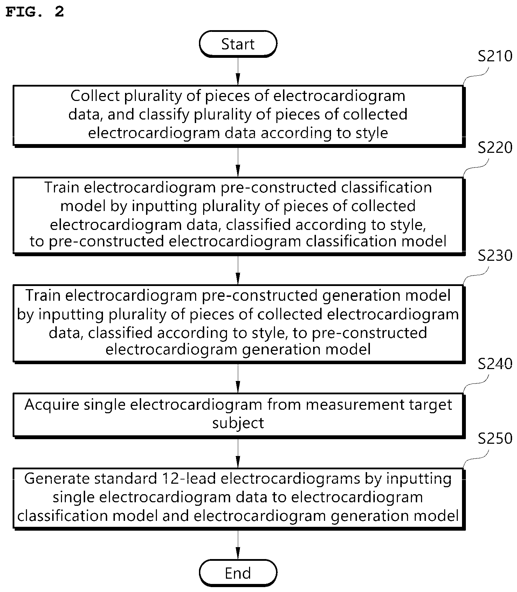

A method for generating a plurality of pieces of standard electrocardiogram data by using the standard electrocardiogram generation apparatus 100 according to an embodiment of the present invention will be described in detail below by using FIG. 2.

FIG. 2 is a flowchart illustrating the method for generating a plurality of pieces of standard electrocardiogram data by using the standard electrocardiogram generation apparatus according to the embodiment of the present invention.

As shown in FIG. 2, the standard electrocardiogram generation apparatus 100 according to the embodiment of the present invention collects standard electrocardiogram data in step S210.

Additionally, the data collection unit 110 collects standard 12-lead electrocardiograms from a hospital server.

Meanwhile, the data collection unit 110 additionally collects style information indicating the lead type of electrocardiogram to which the electrocardiogram data corresponds, individual information indicating an individual's body and mental conditions, and health condition information in response to the collected electrocardiogram data.

Thereafter, the data collection unit 110 classifies the collected electrocardiogram data according to the type of style thereof.

When step S210 is completed, the first training unit 120 trains the constructed electrocardiogram classification model by inputting the collected electrocardiogram data and the style information corresponding to the collected electrocardiogram data to the constructed electrocardiogram classification model in step S220.

First, the first training unit 120 constructs an electrocardiogram classification model to classify electrocardiogram data according to the style thereof. Then, the first training unit 120 receives electrocardiogram data and style information corresponding to the electrocardiogram data in the constructed electrocardiogram classification model, and extracts the features of the electrocardiogram data using the received style information.

The first training unit 120 trains the electrocardiogram classification model by using the electrocardiogram data as input data and the extracted features as output data.

The second training unit 120 trains the electrocardiogram generation model by inputting the collected electrocardiogram data in step S230.

First, the second training unit 120 constructs a deep learning-based electrocardiogram generation model to be trained on the styles of respective lead electrocardiograms. In this case, a plurality of electrocardiogram generation models may be constructed to correspond to the respective styles. Furthermore, each of the electrocardiogram generation models may be trained using one piece of electrocardiogram data, or may be trained using two or more pieces of electrocardiogram data. That is, each of the electrocardiogram generation models is trained on the style of input electrocardiogram data and then generates input electrocardiogram data into a virtual electrocardiogram using the learned style.

When step S230 is completed, the data input unit 140 acquires a single piece of or two pieces of electrocardiogram data from a first lead electrocardiogram measurement device (not shown) attached to a measurement target subject in step S240.

In this case, when there are a plurality of input electrocardiograms, they may be asynchronous electrocardiogram data.

The electrocardiogram generation unit 150 generates a plurality of pieces of standard electrocardiogram data by using the single piece of or two pieces of input electrocardiogram data in step S250.

More specifically, the electrocardiogram generation unit 150 inputs a single piece of or two pieces of electrocardiogram data to the electrocardiogram classification model. Then, the electrocardiogram classification model determines the lead type of electrocardiogram by analyzing the style of single piece of or two pieces of input electrocardiogram data.

When the analysis is completed, the electrocardiogram generation unit 150 generates standard electrocardiogram data of other electrocardiogram styles exclusive of the determined lead electrocardiogram data by using the electrocardiogram generation model.

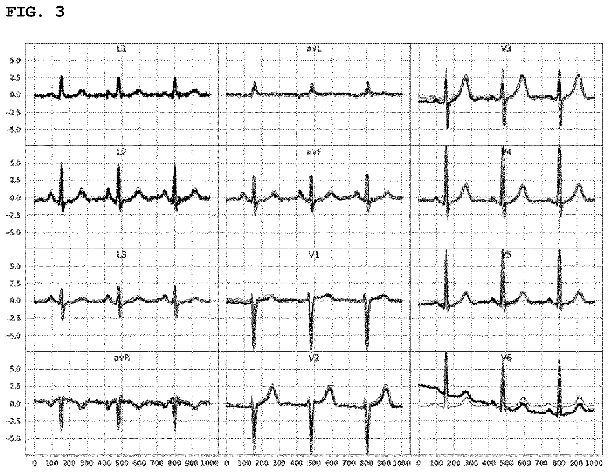

FIG. 3 is an example diagram illustrating step S250 shown in FIG. 2.

As shown in FIG. 3, it is assumed that input electrocardiogram data is V1-lead electrocardiogram data. Then, the electrocardiogram generation unit 150 generates the remaining 11 pieces of standard electrocardiogram data exclusive of the V1-lead electrocardiogram data.

Meanwhile, in the embodiments of the present invention, the lead type of electrocardiogram data to which input electrocardiogram data corresponds is analyzed, and the electrocardiogram classification model and the electrocardiogram generation model are constructed separately in order to generate a plurality of pieces of standard electrocardiogram data according to the results of the analysis. However, the electrocardiogram classification model and the electrocardiogram generation model may be the same learning models, or may be differently constructed learning models.

According to the present invention described above, a plurality of pieces of standard electrocardiogram information are generated from 2-lead electrocardiogram information by using a deep learning algorithm, so that the highly accurate electrocardiograms that are not affected by a measurement target subject's age, gender, presence/absence of disease, and electrode attachment locations can be provided.

While the present invention has been described with reference to the embodiments shown in the drawings, these are merely examples. It will be understood by those skilled in the art that various modifications and other equivalent embodiments may be made therefrom. Therefore, the true technical protection range of the present invention should be determined by the technical spirit of the following patent claims.

## Claims

1. A method for generating a plurality of pieces of standard electrocardiogram data by using a standard electrocardiogram generation apparatus, the method comprising:
collecting standard 12-lead electrocardiogram data, and classifying the collected standard 12-lead electrocardiogram data into a plurality of styles according to characteristics of a measurement target subject and a specific method;
training an electrocardiogram generation model to generate a plurality of lead electrocardiograms by inputting the electrocardiogram data, classified into the plurality of styles, to the electrocardiogram generation model;
receiving single- or 2-lead electrocardiogram data measured from the measurement target subject; and
generating a plurality of lead electrocardiograms by inputting the single- or 2-lead electrocardiogram data to the electrocardiogram generation model.

2. The method of claim 1, further comprising training an electrocardiogram classification model to determine a lead type of electrocardiogram to which input electrocardiogram data corresponds by inputting the electrocardiogram data, classified into the plurality of styles, to the electrocardiogram classification model.

3. The method of claim 1, wherein classifying the collected standard 12-lead electrocardiogram data into the plurality of styles comprises collecting electrocardiogram data, style information indicating a lead type of electrocardiogram to which the electrocardiogram data corresponds, individual information indicating an individual's body and mental conditions, and health condition information.

4. The method of claim 2, wherein training the electrocardiogram classification model to determine the lead type of electrocardiogram comprises extracting features of lead electrocardiograms by using a plurality of pieces of classified electrocardiogram data and style information corresponding to the plurality of pieces of classified electrocardiogram data, and then training the electrocardiogram classification model by using the electrocardiogram data as input data and the extracted features as output data.

5. The method of claim 4, wherein generating the plurality of lead electrocardiograms comprises constructing an electrocardiogram generation model obtained by mixing a generative adversarial network and an autoencoder method, and training the constructed electrocardiogram generation model by conditionally inputting random variables and indices for electrocardiograms to the constructed electrocardiogram generation model.

6. The method of claim 1, wherein generating the plurality of lead electrocardiograms comprises extracting features of input electrocardiogram data by inputting input electrocardiogram data or 2-lead electrocardiogram data to the electrocardiogram classification model, and determining which of standard 12-lead electrocardiograms is the input lead electrocardiogram data or 2-lead electrocardiogram data according to the extracted features.

7. The method of claim 6, wherein generating the plurality of lead electrocardiograms comprises generating a plurality of electrocardiograms of remaining styles exclusive of a corresponding lead electrocardiogram by inputting results of the determination and lead electrocardiogram data or 2-lead electrocardiogram data to the electrocardiogram generation model.

8. The method of claim 1 or 2, wherein the electrocardiogram classification model and the electrocardiogram generation model are same learning models or differently constructed learning models.
